# EUROPEAN PATENT SPECIFICATION

(11) **EP 2 635 207 B1**
(45) Date of publication and mention of the grant of the patent: **17.01.2018**
(21) Application number: 11784887.9
(22) Date of filing: 04.11.2011
(51) Int. Cl.: H02J 7/00, A61B 18/00, A61B 18/04, A61B 18/14, A61B 17/32, A61B 18/12, A61B 17/29, A61B 90/40, A61B 17/064, A61B 17/00, A61B 17/28, A61B 17/285, A61B 90/00

(54) **MEDICAL DEVICE USAGE DATA PROCESSING**
NUTZUNGSDATENVERARBEITUNG BEI EINER MEDIZINISCHEN VORRICHTUNG
TRAITEMENT DES DONNÉES D'UTILISATION D'UN DISPOSITIF MÉDICAL

(30) Priority: 05.11.2010 US 410603 P; 19.10.2011 US 201113276725; 19.05.2011 US 201161487846 P
(43) Date of publication of application: 11.09.2013
(73) Proprietor: Ethicon LLC, 00969 Guaynabo (PR)
(72) Inventor: HOUSER, Kevin L., Springboro, OH 45066 (US); STULEN, Foster B., Mason, OH 45040 (US); DIETZ, Timothy G., Wayne, PA 19087 (US); WILLIS, John W., Cincinnati, OH 45244 (US); KORVICK, Donna L., Maineville, OH 45039 (US); MADAN, Ashvani Kumar, Mason, OH 45040 (US); ZINGMAN, Aron O., Cambridge, MA 02139 (US)
(74) Representative: Tunstall, Christopher Stephen
(86) International application number: PCT/US2011/059378
(87) International publication number: WO 2012/061737

(56) References cited:
- EP-A1- 1 330 991
- EP-A2- 1 997 439
- WO-A1-2011/089270
- WO-A2-2008/102154
- US-A1- 2002 193 709
- US-A1- 2009 281 464

## Description

### PRIORITY

This application claims priority to U.S. Provisional Application Serial No. 61/410,603, filed November 5, 2010, entitled "Energy-Based Surgical Instruments," the disclosure of which is incorporated by reference herein.

This application also claims priority to U.S. Provisional Application Serial No. 61/487,846, filed May 19, 2011, entitled "Energy-Based Surgical Instruments," the disclosure of which is incorporated by reference herein.

This application also claims priority to U.S. Nonprovisional Application Serial No. 13/276,725, filed October 19, 2011, entitled "MEDICAL DEVICE USAGE DATA PROCESSING," the disclosure of which is incorporated by reference herein.

### BACKGROUND

In some settings, endoscopic surgical instruments may be preferred over traditional open surgical devices since a smaller incision may reduce the post-operative recovery time and complications. Consequently, some endoscopic surgical instruments may be suitable for placement of a distal end effector at a desired surgical site through a cannula of a trocar. These distal end effectors may engage tissue in a number of ways to achieve a diagnostic or therapeutic effect (e.g., endocutter, grasper, cutter, stapler, clip applier, access device, drug/gene therapy delivery device, and energy delivery device using ultrasound, RF, laser, etc.). Endoscopic surgical instruments may include a shaft between the end effector and a handle portion, which is manipulated by the clinician. Such a shaft may enable insertion to a desired depth and rotation about the longitudinal axis of the shaft, thereby facilitating positioning of the end effector within the patient.

Examples of endoscopic surgical instruments include those disclosed in U.S. Pat. Pub. No. 2006/0079874, entitled "Tissue Pad Use with an Ultrasonic Surgical Instrument," published April 13, 2006, the disclosure of which is incorporated by reference herein; U.S. Pat. Pub. No. 2007/0191713, entitled "Ultrasonic Device for Cutting and Coagulating," published August 16, 2007, the disclosure of which is incorporated by reference herein; U.S. Pat. Pub. No. 2007/0282333, entitled "Ultrasonic Waveguide and Blade," published December 6, 2007, the disclosure of which is incorporated by reference herein; U.S. Pat. Pub. No. 2008/0200940, entitled "Ultrasonic Device for Cutting and Coagulating," published August 21, 2008, the disclosure of which is incorporated by reference herein; U.S. Pub. No. 2009/0209990 entitled "Motorized Surgical Cutting and Fastening Instrument Having Handle Based Power Source," published August 20, 2009, the disclosure of which is incorporated by reference herein; and U.S. Pub. No. 2010/0069940 entitled "Ultrasonic Device for Fingertip Control," published March 18, 2010, the disclosure of which is incorporated by reference herein; U.S. Pat. Pub. No. 2011/0015660, entitled "Rotating Transducer Mount for Ultrasonic Surgical Instruments," published January 20, 2011, the disclosure of which is incorporated by reference herein; U.S. Pat. No. 6,500,176, entitled "Electrosurgical Systems and Techniques for Sealing Tissue," issued December 31, 2002, the disclosure of which is incorporated by reference herein; U.S. Pat. No. 7,416,101 entitled "Motor-Driven Surgical Cutting and Fastening Instrument with Loading Force Feedback," issued August 26, 2008, the disclosure of which is incorporated by reference herein; U.S. Pat. No. 7,738,971 entitled "Post-Sterilization Programming of Surgical Instruments," issued June 15, 2010, the disclosure of which is incorporated by reference herein; and U.S. Pat. Pub. No. 2011/0087218, entitled "Surgical Instrument Comprising First and Second Drive Systems Actuatable by a Common Trigger Mechanism," published April 14, 2011, the disclosure of which is incorporated by reference herein. Additionally, such surgical tools may include a cordless transducer such as that disclosed in U.S. Pat. Pub. No. 2009/0143797, entitled "Cordless Hand-held Ultrasonic Cautery Cutting Device," published June 4, 2009, the disclosure of which is incorporated by reference herein. In addition, the surgical instruments may be used, or adapted for use, in robotic-assisted surgery settings such as that disclosed in U.S. Pat. No. 6,783,524, entitled "Robotic Surgical Tool with Ultrasound Cauterizing and Cutting Instrument," issued August 31, 2004.
EP 1997439 A2 discusses a pneumatically powered surgical cutting and fastening instrument with electrical feedback. The instrument may include a memory device including a removable medium, for storing or recording a state of the system.

While several systems and methods have been made and used for surgical instruments, it is believed that no one prior to the inventors has made or used the invention described in the appended claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a schematic view of an exemplary medical device having an internal power source;
FIG. 2 depicts a perspective view of an exemplary medical device having an internal power source;
FIG. 3 depicts a side elevational view of the exemplary surgical instrument of with an exemplary memory card;
FIG. 4 depicts a perspective view of an exemplary card reader connected via a wired connection to a PC, the card reader configured to receive and read the exemplary memory card of FIG. 3;
FIG. 5 depicts a perspective view of an exemplary surgical instrument communicating wirelessly with an exemplary base station;
FIG. 6 depicts a cross-sectional end view of the exemplary surgical instrument of FIG. 3 taken along line 6-6 of FIG. 3;
FIG. 7 depicts an alternative cross-sectional view of the exemplary surgical instrument of FIG. 3;
FIG. 8 depicts a side elevational view of an exemplary surgical instrument with an exemplary memory card and battery pack assembly;
FIG. 9 depicts a perspective view of an exemplary battery pack charger and card reader device connected to various other devices;
FIG. 10 depicts a perspective view of an exemplary electrosurgical medical device with an exemplary end effector of the device being tested in a saline solution;
FIG. 11 depicts a perspective view of the end effector of FIG. 10 being tested on a tissue proxy; and
FIG. 12 depicts an exemplary graph illustrating the differences between impedance over time for an exemplary electrosurgical medical device in each of a non-functioning and a functioning state.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

### I. Medical Devices for Use With Insertable or Reclaimable Components

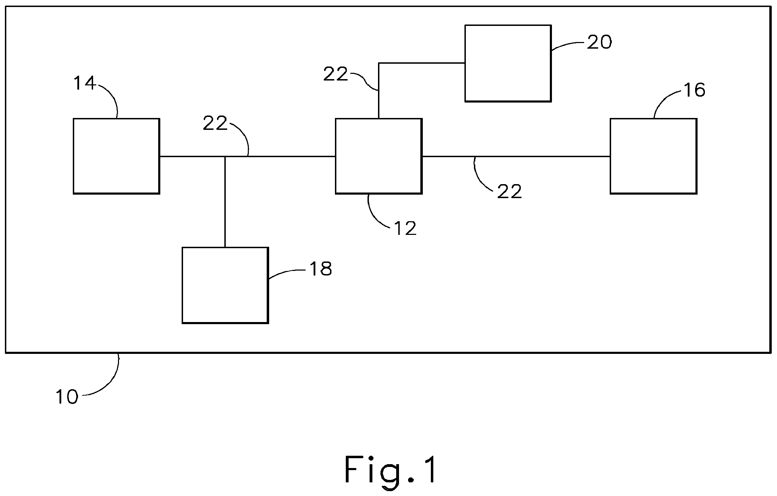

FIG. 1 shows components of an exemplary medical device (10) in diagrammatic block form. As shown, medical device (10) comprises a control module (12), a power source (14), and an end effector (16). Merely exemplary power sources (14) may include NiMH batteries, Li-ion batteries (e.g., prismatic cell type lithium ion batteries, etc.), Ni-Cad batteries, or any other type of power source as may be apparent to one of ordinary skill in the art in light of the teachings herein. Control module (12) may comprise a microprocessor, an application specific integrated circuit (ASIC), memory, a printed circuit board (PCB), a storage device (such as a solid state drive or hard disk), firmware, software, or any other suitable control module components as will be apparent to one of ordinary skill in the art in light of the teachings herein. Control module (12) and power source (14) are coupled by an electrical connection (22), such as a cable and/or traces in a circuit board, etc., to transfer power from power source (14) to control module (12). Alternatively, power source (14) may be selectively coupled to control module (12). This allows power source (14) to be detached and removed from medical device (10), which may further allow power source (14) to be readily recharged or reclaimed for resterilization and reuse, such as in accordance with the various teachings herein. In addition or in the alternative, control module (12) may be removed for servicing, testing, replacement, or any other purpose as will be apparent to one of ordinary skill in the art in view of the teachings herein.

End effector (16) is coupled to control module (12) by another electrical connection (22). End effector (16) is configured to perform a desired function of medical device (10). By way of example only, such function may include cauterizing tissue, ablating tissue, severing tissue, ultrasonically vibrating, stapling tissue, or any other desired task for medical device (10). End effector (16) may thus include an active feature such as an ultrasonic blade, a pair of clamping jaws, a sharp knife, a staple driving assembly, a monopolar RF electrode, a pair of bipolar RF electrodes, a thermal heating element, and/or various other components. End effector (16) may also be removable from medical device (10) for servicing, testing, replacement, or any other purpose as will be apparent to one of ordinary skill in the art in view of the teachings herein. In some versions, end effector (16) is modular such that medical device (10) may be used with different kinds of end effectors (e.g., as taught in U.S. Provisional Application Serial No. 61/410,603, etc.). Various other configurations of end effector (16) may be provided for a variety of different functions depending upon the purpose of medical device (10) as will be apparent to those of ordinary skill in the art in view of the teachings herein. Similarly, other types of components of a medical device (10) that may receive power from power source (14) will be apparent to those of ordinary skill in the art in view of the teachings herein.

Medical device (10) of the present example includes a trigger (18) and a sensor (20), though it should be understood that such components are merely optional. Trigger (18) is coupled to control module (12) and power source (14) by electrical connection (22). Trigger (18) may be configured to selectively provide power from power source (14) to end effector (16) (and/or to some other component of medical device (10)) to activate medical device (10) when performing a procedure. Sensor (20) is also coupled to control module (12) by an electrical connection (22) and may be configured to provide a variety of information to control module (12) during a procedure. By way of example only, such configurations may include sensing a temperature at end effector (16) or determining the oscillation rate of end effector (16). Data from sensor (20) may be processed by control module (12) to effect the delivery of power to end effector (16) (e.g., in a feedback loop, etc.). Various other configurations of sensor (20) may be provided depending upon the purpose of medical device (10) as will be apparent to those of ordinary skill in the art in view of the teachings herein. Of course, as with other components described herein, medical device (10) may have more than one sensor (20), or sensor (20) may simply be omitted if desired.

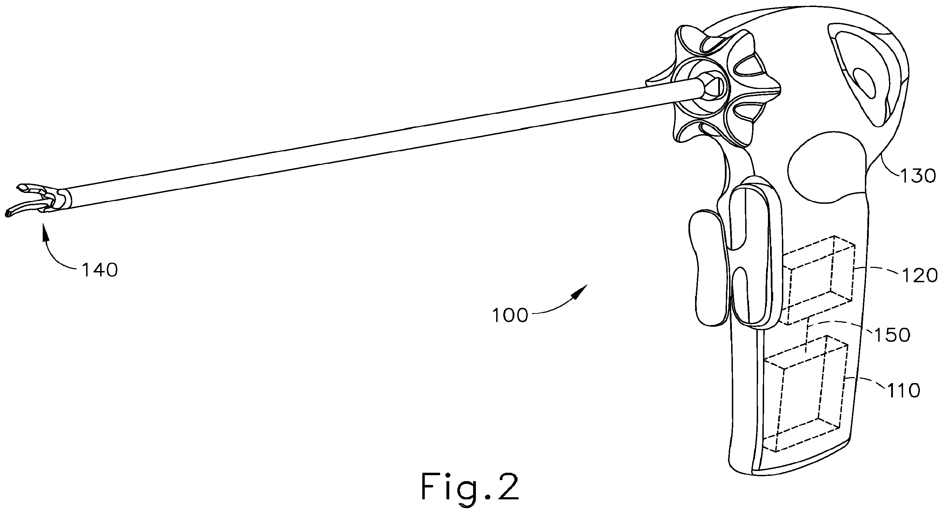

FIG. 2 depicts a merely exemplary form that medical device (10) may take. In particular, FIG. 2 shows a medical device (100) comprising a power source (110), a control module (120), a housing (130), end effector (140), and an electrical connection (150). In the present example, power source (110) is located internally within housing (130) of medical device (100). Alternatively, power source (110) may only partially extend into housing (130) and may be selectively attachable to a portion of housing (130). In yet a further exemplary configuration, a portion of housing (130) may extend into power source (110) and power source (110) may be selectively attachable to the portion of housing (130). Power source (110) may also be configured to detach from medical device (100) and decouple from control module (120) or electrical connection (150). As a result, power source (110) may be completely separated from medical device (100) in some versions. By way of example only, power source (110) may be constructed in accordance with the teachings of U.S. Pub. No. 2011/0087212, entitled "Surgical Generator for Ultrasonic and Electrosurgical Devices," published April 14, 2011, the disclosure of which is incorporated by reference herein. In some versions, power source (110) may be removed to be recharged or reclaimed for resterilization and reuse, such as in accordance with various teachings herein. After recharging, or after an initial charge, power source (110) may be inserted or reinserted into medical device (100) and secured to housing (130) or internally within housing (130). Of course, medical device (100) may also allow power source (110) to be charged and/or recharged while power source (110) is still in or otherwise coupled relative to housing (130).

It should also be understood that control module (120) may be removed for servicing, testing, replacement, or any other purpose as will be apparent to one of ordinary skill in the art in view of the teachings herein. Further, end effector (140) may also be removable from medical device (100) for servicing, testing, replacement, or any other purpose as will be apparent to one of ordinary skill in the art in view of the teachings herein.

While certain configurations of an exemplary medical device (100) have been described, various other ways in which medical device (100) may be configured will be apparent to those of ordinary skill in the art in view of the teachings herein. By way of example only, medical devices (10, 100) and/or any other medical device referred to herein may be constructed in accordance with at least some of the teachings of U.S. Pat. No. 5,322,055 entitled "Clamp Coagulator/Cutting System for Ultrasonic Surgical Instruments," issued June 21, 1994, the disclosure of which is incorporated by reference herein; U.S. Pat. No. 5,873,873 entitled "Ultrasonic Clamp Coagulator Apparatus Having Improved Clamp Mechanism," issued February 23, 1999, the disclosure of which is incorporated by reference herein; U.S. Pat. No. 5,980,510, entitled "Ultrasonic Clamp Coagulator Apparatus Having Improved Clamp Arm Pivot Mount," filed October 10, 1997, the disclosure of which is incorporated by reference herein; U.S. Pat. No. 6,325,811 entitled "Blades with Functional Balance Asymmetries for use with Ultrasonic Surgical Instruments," issued December 4, 2001, the disclosure of which is incorporated by reference herein; U.S. Pub. No. 2006/0079874 entitled "Tissue Pad for Use with an Ultrasonic Surgical Instrument," published April 13, 2006, the disclosure of which is incorporated by reference herein; U.S. Pub. No. 2007/0191713 entitled "Ultrasonic Device for Cutting and Coagulating," published August 16, 2007, the disclosure of which is incorporated by reference herein; U.S. Pub. No. 2007/0282333 entitled "Ultrasonic Waveguide and Blade," published December 6, 2007, the disclosure of which is incorporated by reference herein; U.S. Pub. No. 2008/0200940 entitled "Ultrasonic Device for Cutting and Coagulating," published August 21, 2008, the disclosure of which is incorporated by reference herein; U.S. Pub. No. 2009/0143797, entitled "Cordless Hand-held Ultrasonic Cautery Cutting Device," published June 4, 2009, the disclosure of which is incorporated by reference herein; U.S. Pub. No. 2010/0069940 entitled "Ultrasonic Device for Fingertip Control," published March 18, 2010, the disclosure of which is incorporated by reference herein; U.S. Pub. No. 2011/0015660, entitled "Rotating Transducer Mount for Ultrasonic Surgical Instruments," published January 20, 2011, the disclosure of which is incorporated by reference herein; and/or U.S. Provisional Application Serial No. 61/410,603, filed November 5, 2010, entitled "Energy-Based Surgical Instruments," the disclosure of which is incorporated by reference herein.

Of course, housing (130) and medical device (100) may include other configurations. For instance, housing (130) and/or medical device (100) may include a tissue cutting element and one or more elements that transmit bipolar RF energy to tissue (e.g., to coagulate or seal the tissue). An example of such a device is the ENSEAL® Tissue Sealing Device by Ethicon Endo-Surgery, Inc., of Cincinnati, Ohio. Further examples of such devices and related concepts are disclosed in U.S. Pat. No. 6,500,176 entitled "Electrosurgical Systems and Techniques for Sealing Tissue," issued December 31, 2002, the disclosure of which is incorporated by reference herein; U.S. Pat. No. 7,112,201, entitled "Electrosurgical Instrument and Method of Use," issued September 26, 2006, the disclosure of which is incorporated by reference herein; U.S. Pat. No. 7,125,409, entitled "Electrosurgical Working End for Controlled Energy Delivery," issued October 24, 2006, the disclosure of which is incorporated by reference herein; U.S. Pat. No. 7,169,146 entitled "Electrosurgical Probe and Method of Use," issued January 30, 2007, the disclosure of which is incorporated by reference herein; U.S. Pat. No. 7,186,253, entitled "Electrosurgical Jaw Structure for Controlled Energy Delivery," issued March 6, 2007, the disclosure of which is incorporated by reference herein; U.S. Pat. No. 7,189,233, entitled "Electrosurgical Instrument," issued March 13, 2007, the disclosure of which is incorporated by reference herein; U.S. Pat. No. 7,220,951, entitled "Surgical Sealing Surfaces and Methods of Use," issued May 22, 2007, the disclosure of which is incorporated by reference herein; U.S. Pat. No. 7,309,849, entitled "Polymer Compositions Exhibiting a PTC Property and Methods of Fabrication," issued December 18, 2007, the disclosure of which is incorporated by reference herein; U.S. Pat. No. 7,311,709, entitled "Electrosurgical Instrument and Method of Use," issued December 25, 2007, the disclosure of which is incorporated by reference herein; U.S. Pat. No. 7,354,440, entitled "Electrosurgical Instrument and Method of Use," issued April 8, 2008, the disclosure of which is incorporated by reference herein; U.S. Pat. No. 7,381,209, entitled "Electrosurgical Instrument," issued June 3, 2008, the disclosure of which is incorporated by reference herein; U.S. Pub. No. 2011/0087218, entitled "Surgical Instrument Comprising First and Second Drive Systems Actuatable by a Common Trigger Mechanism," published April 14, 2011, the disclosure of which is incorporated by reference herein; and U.S. Pat. App. No. 13/151,181, entitled "Motor Driven Electrosurgical Device with Mechanical and Electrical Feedback," filed June 2, 2011, the disclosure of which is incorporated by reference herein.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

### II. Exemplary Restriction and Tracking of Use of a Surgical Instrument

Examples described below relate to various components and arrangements for restricting and/or tracking use of surgical instruments. Such components and arrangements may be used with ultrasonic surgical instruments, RF electrosurgical instruments, and/or various other kinds of surgical instruments. By way of example only, the teachings below may be readily incorporated into the surgical instruments or devices (10, 100) described above; and/or into the various instruments described in the various references cited herein. Other suitable combinations will be apparent to those of ordinary skill in the art in view of the teachings herein.

### A. Exemplary Memory Card

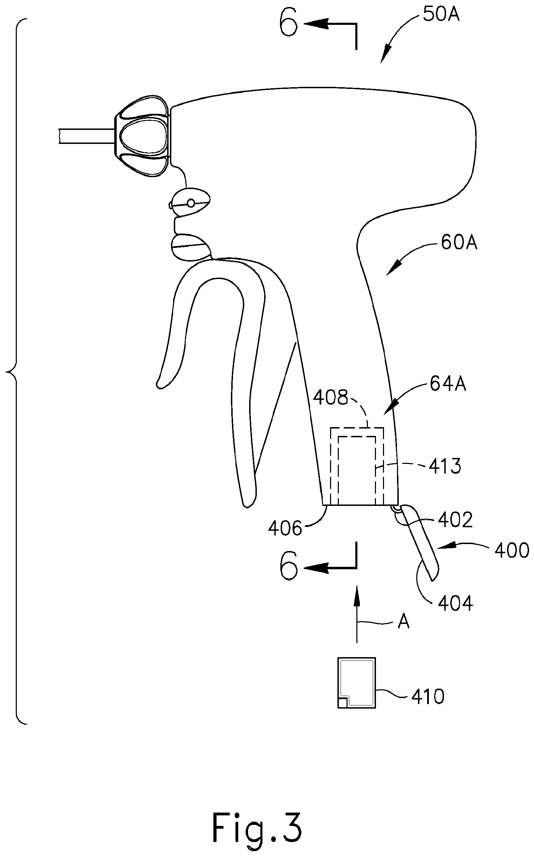

FIG. 3 shows an exemplary surgical instrument (50A) that includes lower portion (64A). It should be understood that instrument (50A) may comprise a modified version of instrument (10, 100) described above. Lower portion (64A) includes end piece (400) hingedly connected to lower portion (64A) of multi-piece handle assembly (60A) via hinge (402). In a closed position, upper surface (404) of end piece (400) abuts lower surface (406) of lower portion (64A) to seal end piece (400) to lower portion (64A). A fastening assembly such as a latch or pin and slot assembly may be used to connect end piece (400) to lower portion (64A). In an open position, and as shown in FIG. 3, end piece (400) is pivoted away from multi-piece handle assembly (60A) about hinge (402). Aperture (408) is defined in surgical instrument (50A) and is configured to receive data card (410) when end piece (400) is in the open position. For example, data card (410) is inserted into aperture (408) in the direction of arrow (A).

Data card (410) may comprise a memory card (e.g., Flash memory, etc.) or sim card operable to store information, as discussed below. In use, a clean data card (410) is loaded into electronics module (413), described below. In some versions, electronics module (413) comprises a battery pack including a slot or aperture to receive data card (410), as described below. Data card (410) may control and include information regarding instrument (50A) such as nominal frequency and a device identifier; and may include a generator control algorithm to adjust amounts of energy being received from a generator. Data card (410) carries different control algorithm values that may affect anything from the basic operation of instrument (50A) to advanced vessel sealing for larger vessels worked on with an end effector of instrument (50A). After a use, data card (410) may be inactivated. A new data card (410) may be required for new and refurbished disposable electronics modules (413) within multi-piece handle assembly (60A).

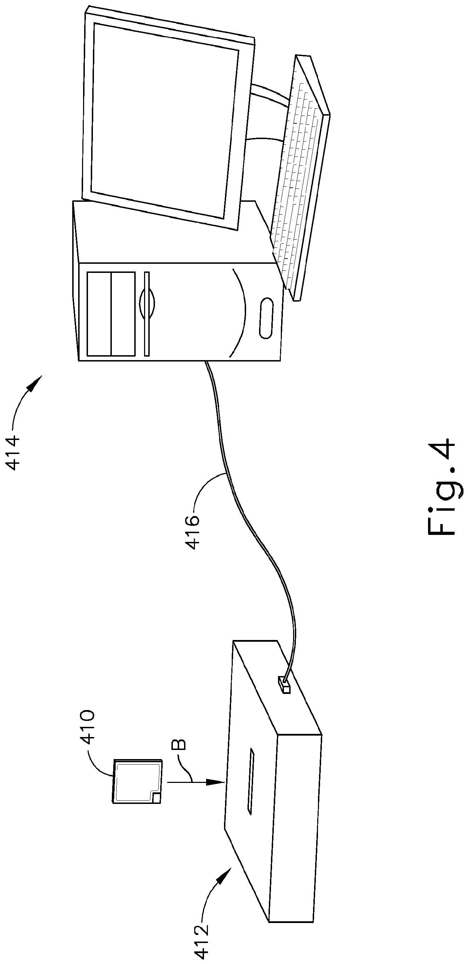

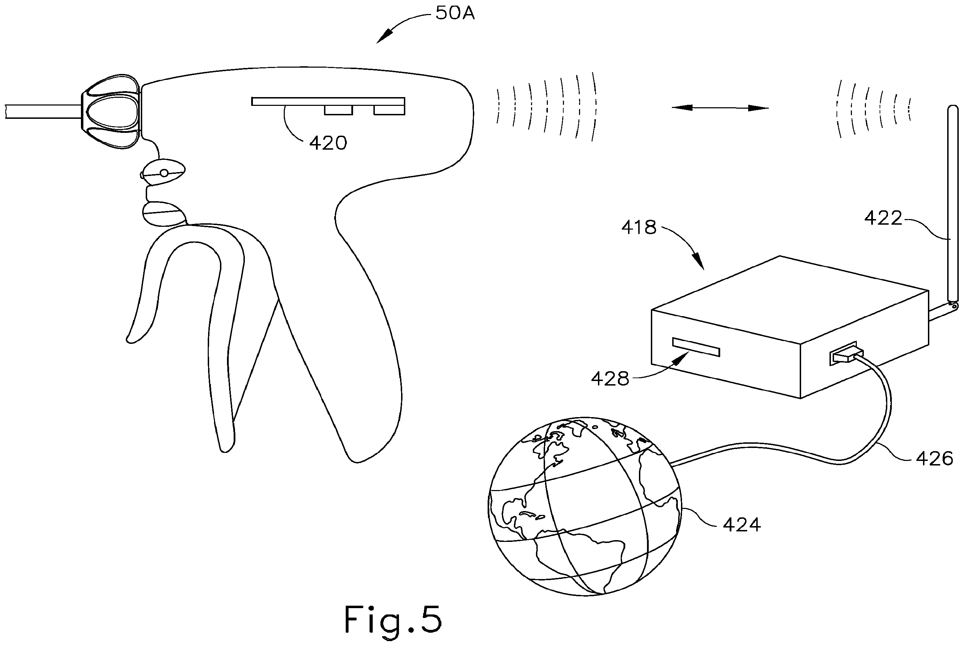

As shown in FIG. 4, data card (410) is also insertable into a card reader (412) in the direction of arrow (B). FIG. 4 shows card reader (412) connected to a computer, such as PC (414), via a wired connection through cable (416). Of course, card reader (412) may take a variety of other forms as will be apparent to those of ordinary skill in the art in view of the teachings herein. PC (414) may be connected to a network or to World Wide Web (424) (FIG. 5). Information about the use of surgical instrument (50A) stored in data card (410) loaded within electronics module (413) during use of instrument (50A) can thereby be transmitted for use in diagnostics or for purposes of billing for the use of electronics module (413) during the procedure. Alternatively, when PC (414) is connected to a network or World Wide Web (424), the time of use of instrument (50A) is transmitted to the manufacturer of electronics module (413) via the connection to allow for a fee for use to be based on the usage time of instrument (50A) as opposed to charging for instrument (50A) itself. Data card (410) could be used to purchase usage time from the manufacturer. Data card (410) would then be inserted into electronics module (413) and would allow instrument (50A) to function for the prepaid number of minutes.

Alternatively, data from data card (410) may be wirelessly communicated to base station (418) as shown in FIG. 5. Wireless communications board (420) housed in multi-piece handle assembly (60A) of surgical instrument (50A) communicates information via a two-way link with base station (418). Such information may be received, and/or information may be sent, from antenna (422) of base station (418). Base station (418) is connected to World Wide Web (424) and/or some other network via conduit (426), for example, to further communicate the information to outside sources, such as a manufacturer. Data card (410) may additionally be received within card reader slot (428) of base station (418) to download information from or upload information to data card (410).

A previous business model has included selling surgical instrument for use. It may be desirable for the user to pay for use of the instrument instead of paying for the instrument itself. This would allow use of the device in a short or long procedure to be charged differently based on the length of the procedure. More flexibility is thus allowed in pricing for individual users of the instrument depending on their requirements of use. While a generator or other piece of capital equipment coupled with a surgical instrument may be polled to determine total use that is chargeable, data card (410) allows for an alternative method of charging for use based on the total time an instrument is used, as described above. Data card (410) may also act to load the device or instrument having a circuit with a prepaid number of minutes, and the instrument may be usable for the prepaid amount of time.

In use, prior to surgery, a user would load data card (410) with a set number of minutes for use, for example. Such a loading for data card (410) may be accomplished through a connection to card reader (412) connected to PC (414), as described above. The same PC (414) may be used to order and/or pay for the requested number of minutes. After the minutes information has been loaded into data card (410), data card (410) may be placed aseptically into aperture (408) of instrument (50A), for example. Instrument (50A) during use would initially read data card (410) to check for the presence of any number of prepaid minutes loaded for use and to determine the number of minutes available for use with instrument (50A). After the procedure has been completed with instrument (50A), a user may remove data card (410) from instrument (50A) and place data card (410) back in card reader (412) to receive credit for any unused prepaid minutes.

Additionally or alternatively, a user may be able to purchase use via data card (410) based on a type of procedure. For example, a short procedure may be charged a first price, and a more complex and/or longer procedure may be charged a different (e.g., higher) second price. Pricing may be determined based on the complexity of the procedure (e.g., regardless of the length of the procedure) and/or based on other criteria. In the above examples, more functionality may be provided for a longer and/or more complex procedure to justify the higher additional cost over a shorter and/or less complex procedure. Such functionality may include, for example, advanced diagnostics and algorithms downloaded to instrument (50A) via data card (410) to assist the user when the longer and/or more complex procedure pricing is purchased. Other suitable pricing structures will be apparent to those of ordinary skill in the art in view of the teachings herein.

In some versions, a RF transmitter may be contained within instrument (50A) to receive and transmit the minutes information such that instrument (50A) may maintain a wireless connection with base station (418), described above, through an entire procedure. Loss of the wireless link may disable instrument (50A), though instrument (50A) may include a backup functionality. Some users may rather select an option of storing use on data card (410) and later being charged for the amount of time instrument (50A) was used during a procedure (e.g., after expiration of the prepaid time, etc.). For example, the stored amount of time could later be read from data card (410) after the procedure via connections described above.

For those users that have purchased an amount of minutes and have lost a wireless connection, instrument (50A) may indicate that the wireless signal was lost. A user would then be aware that the parameters associated with instrument (50A) that were being wirelessly sent and recorded on another device such as base station (418) are no longer being stored. So that a user may continue with a surgery after losing a wireless connection, a first price may be charged for actual use during a wireless session with a steady connection and a second, higher price may be charged for a continued use after the connection was lost. Additionally or alternatively, a backup download ability may be provided on a device such as a Universal Serial Bus ("USB") or data card (410) or other suitable device(s). Such a backup device would allow a user to continue downloading to the backup device to monitor use of instrument (50A) after losing the wireless connection, allowing the user to avoid paying the higher lost connection charge.

Referring back to a steady wireless connection, the total use of instrument (50A) may be transmitted to base station (418) that may be polled to determine an overall use (the amount of time used) of the instrument (50A). In versions of instrument (50A) that provide modular end effectors, data on data card (410) may be configured to discriminate among such various end effectors. Data card (410) may thus store prepaid minutes and/or usage data, among other things, based on the type of end effector utilized with instrument (50A). The end effector utilized may also wirelessly transmit information as described above to instrument (50A), relating to the type and/or form of the utilized end effector.

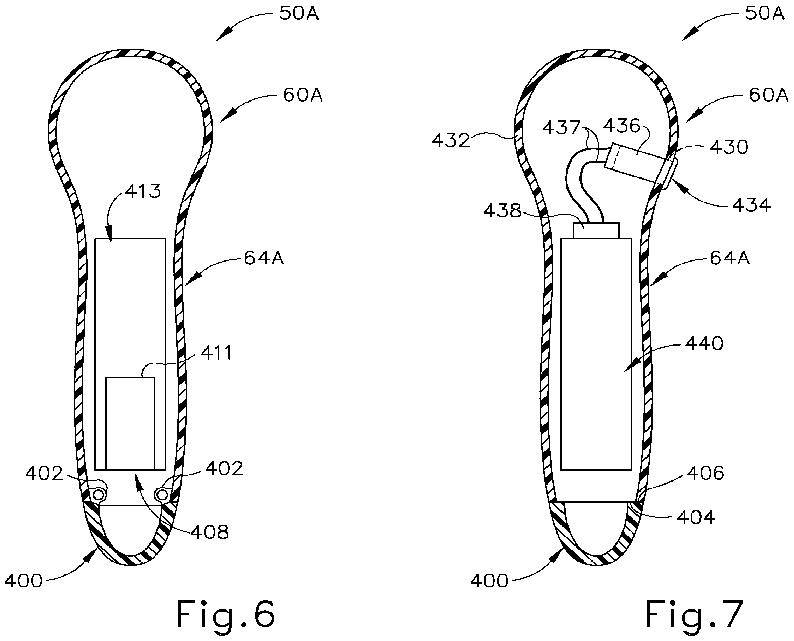

FIG. 6 shows a cross-sectional view of instrument (50A) taken from a proximal to distal perspective. FIG. 3 shows end piece (400) in an open position and swung away from multi-piece handle assembly (60A) about hinge (402), such that aperture (408) is exposed and able to receive data card (410). FIG. 6 shows end piece (400) in a closed position. Aperture (408) shown in FIG. 6 is configured to receive a sterile load. Aperture (408) of the present example is part of electronics board (411) of electronics module (413) disposed within instrument (50A). Insertion of data card (410) into aperture (408) of electronics board (411) electronically connects data card (410) to electronics board (411). Electronics board (411) recognizes the presence of data card (410) and can read or write information to data card (410). In reading from data card (410), electronics board (411) can update its software for improved performance. Alternatively, instrument (50A) may initially only include programming sufficient to read data card (410). The act of loading data card (410) into aperture (408) of electronics board (411) may cause instrument (50A) to read data card (410) and download a full operating system and functional programming from data card (410). Additionally or alternatively, an end effector of instrument (50A) may include a chip that communicates with instrument (50A) to download the operating system and functional programming to instrument (50A) when the end effector is attached to instrument (50A). Such a chip may be used in addition to or in lieu of data card (410).

In writing to data card (410), electronics board (411) can write information pertaining to, among other things, instrument performance, battery charge status, error codes, battery life, number of instrument uses, number of activations during the current use, power curve profiles or other parameters. Electronics board (411) can also write the performance parameters such as current and voltage supplied to either a transducer in an ultrasonic device or the end effector in an electrosurgical device throughout the entire procedure. At the end of the procedure, a user can remove data card (410) from electronics module (413) and attach it to card reader (412) that is positioned on and/or communicates with a computer, such as PC (414) of FIG. 4, or some other storage device such as base station (418) of FIG. 5.

FIG. 7 shows an alternative cross-sectional view of instrument (50A) taken from a proximal to distal perspective. End piece (400) is in a closed position with respect to multi-piece handle assembly (60A) such that upper surface (404) of end piece (400) abuts and is attached to lower surface (406) of lower portion (64A). Slot (430) is provided in one of sidewall portions (432) of multi-piece handle assembly (60A). Slot (430) is configured to receive data card (410). Slot (430) may further include rubber seal (434) disposed over slot (430) to seal slot (430) when data card (410) is received in slot (430). The walls defining slot (430) are part of electronics board (436), which is connected via wires (437) to top surface communications box (438) of electronics module (440). Other than the manner of connection, electronics module (440) and electronics board (436) operate in a similar manner described above for electronics board (411) and electronics module (413). Slot (430) may be sterile prior to loading of data card (410). Additionally or alternatively, data card (410) may be sterile or clean but encased in a sterile dispenser stick delivered into slot (430).

### B. Exemplary Data Card and Battery Pack Assembly

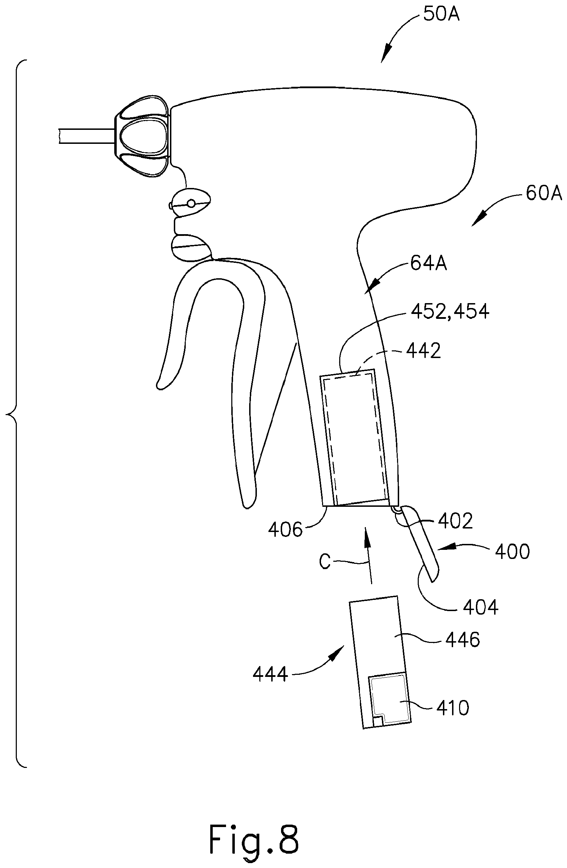

FIG. 8 shows instrument (50A) including aperture (442) configured to receive battery pack and data card assembly (444), which may be inserted into aperture (442) in the direction of arrow (C). Again, instrument (50A) may be provided as a variation of instrument (10, 100) described above. Battery pack and data card assembly (444) includes rechargeable battery (446) and data card (410) attached to battery (446). Data card (410) may either be removable from or permanently attached to battery (446). Battery pack and data card assembly (444) may be inserted in aperture (442) that is attached to electronics module (452) and electronics board (454), which are similar to electronics board (411) and electronics module (413) described above with respect to a version of instrument (50A) shown in FIG. 6. Data card (410) of this example may also be configured and used in the same way as data card (410) described above in the context of FIGS. 3-6.

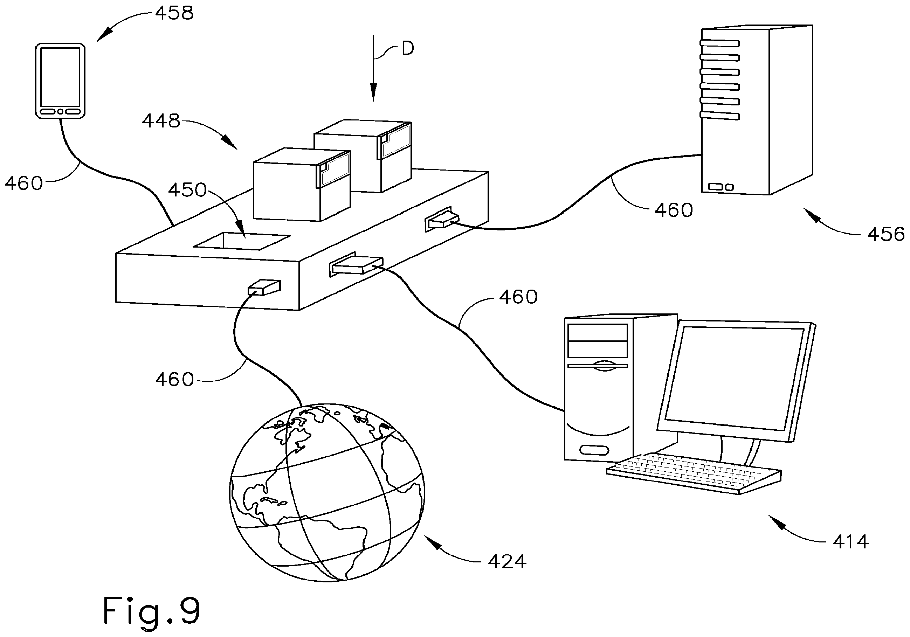

FIG. 9 shows charging station (448) including slots (450), each slot (450) being configured to receive battery pack and data card assembly (444) in the direction of arrow (D). In use, surgical instrument (50A) will record on data card (410) the type of instrument used and an amount of time (measureable in minutes, for example) that instrument (50A) was used for during the procedure. Of course, any other type of data relating to the use and/or operation of instrument (50A) may also be recorded on data card (410). When battery (446) is removed from surgical instrument (50A) and placed on charging station (448), charging station (448) is used to charge rechargeable battery (446). Simultaneously, information from data card (410) would be downloaded and read by charging station (448). The information may be used to determine usage for payment purposes (a customer paying for the amount of time the instrument was used during the procedure). Additionally or alternatively, the information may be relayed to a central storage device that would log a total use of the device(s) in the hospital and/or diagnose problems with instrument (50A), among other possible actions as will be apparent to those of ordinary skill in the art in view of the teachings herein. In addition or in the alternative, the information may include data relating to any errors in the operation of instrument (50A) and/or components of instrument (50A). When charging station (48) is connected to a network of World Wide Web (424), the information may be transmitted to a repair or diagnostic facility or to a manufacturer of instrument (50A) and/or components of instrument (50A).

In use, instrument (50A) may initially read data card (410) not only to check for the presence of a prepaid number of minutes but may also zero out any previous recorded uses. Additionally, data card (410) may also hold information associated with error codes generated during the procedure as well as statistics regarding use, including but not limited to total power consumed by instrument (50A) during the procedure. The information may be sent to at least one of server (456), PC (414), World Wide Web (424) or other network, or a mobile device such as such as smartphone (458), shown as connected to charging station (448) via wires (460). Smartphone (458) may be, but is not limited to being, an iphone®. The mobile device may alternatively be, but is not limited to being, an iPad®. Both iphone® and iPad® are registered trademarks of Apple, Inc. of Cupertino, CA, or a Palm Pre®, a registered trademark of Palm Trademark Holding Company of Sunnyvale, CA, or other similar mobile devices apparent to those of ordinary skill in the art in view of the teachings herein. Software programs can then be used to analyze the data on the memory card for use by the surgeon, the Operation Room ("OR") staff, biomedical researchers, or others.

In another version, electronics module (452) (FIG. 8) would include a wireless communications board, such as wireless communications board (420) shown in FIG. 5, which would be utilize wireless communications such as Bluetooth or any other suitable wireless communications protocol. In this way, electronics module (452) would be able to continually communicate information relative to the use of surgical instrument (50A) or electronics module (452) through a surgical procedure.

### V. Exemplary Testing Feature for Electrosurgical Medical Device

Examples described below relate to a test sequence for electrosurgical devices energized with RF energy, though such testing is possible with other similar devices as will be apparent to those of ordinary skill in the art in view of the teachings herein.

### A. Exemplary Test Sequence

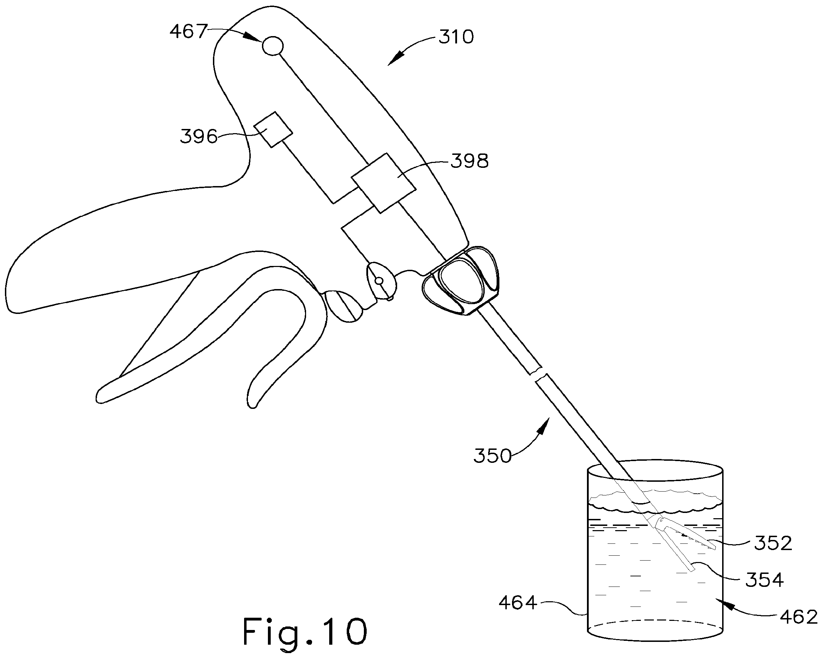

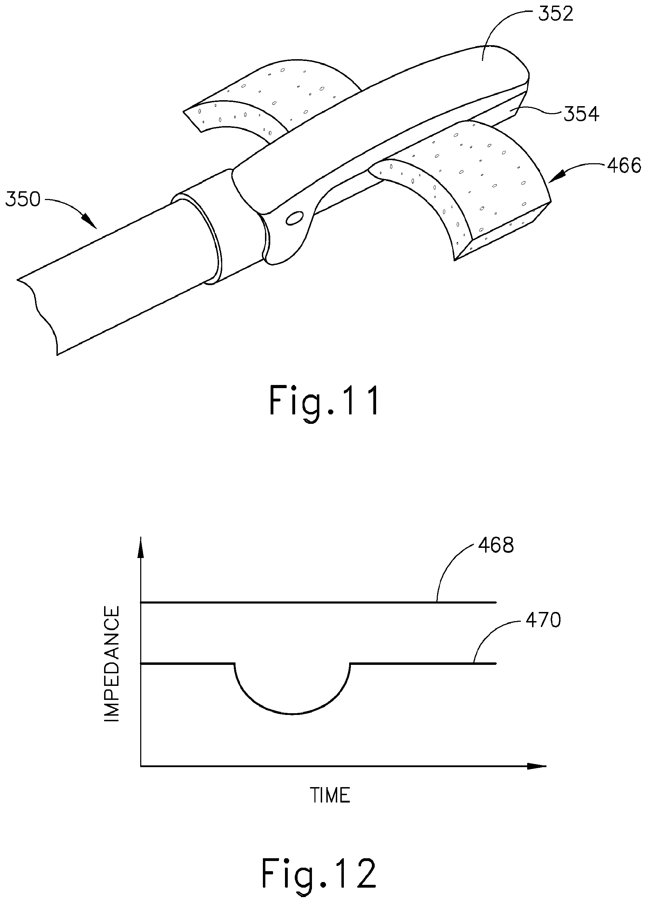

FIGS. 10-11 show use of a testing sequence upon an electrosurgical device (310). Electrosurgical device (310) may be constructed in accordance with any suitable teachings herein and/or any suitable teachings of various references cited herein. In some versions, electrosurgical device (310) includes a motorized knife drive. Examples of such an electrosurgical device are described in U.S. Patent App. No. 13/151,481, entitled "Motor Driven Electrosurgical Device with Mechanical and Electrical Feedback", filed June 2, 2011, the disclosure of which is incorporated by reference herein. In some versions, electrosurgical device (310) will not operate on tissue to sever and seal the tissue unless the test sequence is performed correctly and electrosurgical device (310) passes the test. The test sequence is performed by energizing device (310) in saline (462) contained in glass or container (464), as shown in FIG. 10, or on other tissue proxy such as wet sponge (466) shown in FIG. 11.

Electrode surfaces (not shown) between jaws (352, 354) of device (310) may be used as sensors (e.g., to sense tissue impedance, etc.). Impedance correlates to an amount of resistance to current (such that an increased impedance reduces the flow of current). A power source (396) generates voltage to be sent to jaws (352, 354), and the electrode surfaces act as sensors to sense the impedance. A controller (398) provides an output in response to detected variations in impedance measurements to indicate device (310) is operable. Such output may include illumination of a light (467) disposed on a proximal portion of device (310), an audio output, and/or an output on an attached display screen. Additionally or alternatively, a utilized proxy, as described above, may be included between jaws (352, 354) in a packaging that contains device (310). A user may then activate a test sequence while device (310) and the utilized proxy are still in the packaging and an indicator, as described above, may inform the user about the operability of device (310).

FIG. 12 shows the results for device (310) where device (310) is non-functioning, shown as line (468); and where device (310) is functioning, shown as line (470). A non-functioning device would have the results shown as line (468) such that a continuous amount of impedance would be measured from device (310) over a length of time showing no reaction to an applied amount of voltage. A functioning device would show a reaction to an applied amount of voltage, such as the lowered amount of impedance that varies and oscillates in amount over time as shown by line (470). Particularly, line (470) shows a dip in the levels of impedance measured during the testing sequence, indicating a heightened amount of voltage.

It should be understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

Versions of the devices described above may have application in conventional medical treatments and procedures conducted by a medical professional, as well as application in robotic-assisted medical treatments and procedures.

Versions of described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various versions in the present disclosure, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical instrument comprising:
(a) a battery pack assembly comprising a data card (410) and a battery pack (446);
(b) a body comprising a housing (130) defining an aperture (442), wherein the aperture is configured to receive the battery pack assembly;
(c) a transmission assembly extending distally from the body; and
(d) an end effector (140) at a distal end of the transmission assembly, wherein the end effector is operable to deliver energy from the transmission assembly to a surgical site;
wherein the data card is operable to store information relating to an operating parameter associated with the end effector; and
wherein the housing includes a lower portion and an end piece, wherein the end piece is hingedly attached to the lower portion and is positionable between an open position and a closed position, and wherein the aperture is disposed at a bottom end of the lower portion such that the battery pack assembly is removably received in the aperture when the end piece is in the open position and the battery pack assembly is contained in the aperture when the end piece is in the closed position.

2. The surgical instrument of claim 1, further comprising an electronics module (413), wherein the aperture (442) is configured to receive the electronics module, wherein the battery pack (446) is insertable in the electronics module.

3. The surgical instrument of claim 1, wherein the data card (410) is attached to the battery pack (446).

4. The surgical instrument of claim 1, wherein the data card (410) is configured to be read in a card reader (412) separate from the instrument.

5. The surgical instrument of claim 4, wherein the card reader (412) is in communication with a computer (414) via a wireless connection.

6. The surgical instrument of claim 4, wherein the card reader (412) is configured to transmit a wireless signal including data to a computer (414) when the data card (410) is received in the card reader.

7. The surgical instrument of claim 6, wherein the data comprises a number of minutes the instrument was used during a procedure, further comprising an electronics module (413) in communication with the data card (410), wherein the data is transmittable to a third party associated with the electronics module to bill for use of the electronics module during a procedure.

8. The surgical instrument of claim 6, wherein the data comprises a request to purchase a type of procedure, wherein the type of procedure includes an associated number of minutes and a set of instrument parameters, and wherein the data card (410) is configured to receive information relating to a purchased type of procedure via a purchase transaction, such that the instrument is usable for the associated number of minutes during the purchased type of procedure when the data card is removed from the card reader (412) and received in the aperture of the instrument.

9. The surgical instrument of claim 6, wherein the data comprises a request to purchase a number of minutes, and the data card (410) is configured to receive a purchased number of minutes via a purchase transaction based on the requested number of minutes, such that the instrument is usable for the purchased number of minutes during a procedure when the data card is removed from the card reader (412) and received in the aperture (442) of the instrument.

10. The surgical instrument of claim 9, wherein the computer (414) is configured to send the data to an outside source via a network to conduct the purchase transaction.

11. The surgical instrument of claim 1, wherein the end effector (140) is configured to transmit a wireless signal including data to at least one of a computer (414) or the data card (410).

12. The surgical instrument of claim 1, wherein the data card (410) is configured to transmit a wireless signal including data to a computer (414) when the data card is received in the aperture (442) of the instrument.

## Patentansprüche

1. Chirurgisches Instrument, das Folgendes umfasst:
(a) eine Batteriepaket-Baugruppe, die eine Datenkarte (410) und ein Batteriepaket (446) umfasst,
(b) einen Körper, der ein Gehäuse (130) umfasst, das eine Öffnung (442) definiert, wobei die Öffnung dafür konfiguriert ist, die Batteriepaket-Baugruppe aufzunehmen,
(c) eine Übertragungsbaugruppe, die sich distal von dem Körper erstreckt, und
(d) einen Endeffektor (140) an einem distalen Ende der Übertragungsbaugruppe, wobei der Endeffektor funktionsfähig ist, um Energie von der Übertragungsbaugruppe an eine Operationsstelle zu liefern,
wobei die Datenkarte funktionsfähig ist, um Daten bezüglich eines mit dem Endeffektor verknüpften Betriebsparameters zu speichern, und
wobei das Gehäuse einen unteren Abschnitt und ein Endstück einschließt, wobei das Endstück an dem unteren Abschnitt befestigt ist und zwischen einer offenen Position und einer geschlossenen Position positionierbar ist, und wobei die Öffnung derart an einem unteren Ende des unteren Abschnitts angeordnet ist, dass die Batteriepaket-Baugruppe entfernbar in der Öffnung aufgenommen wird, wenn sich das Endstück in der offenen Position befindet, und die Batteriepaket-Baugruppe in der Öffnung zurückgehalten wird, wenn sich das Endstück in der geschlossenen Position befindet.

2. Chirurgisches Instrument nach Anspruch 1, das ferner ein Elektronikmodul (413) umfasst, wobei die Öffnung (442) dafür konfiguriert ist, das Elektronikmodul aufzunehmen, wobei das Batteriepaket (446) in das Elektronikmodul einsetzbar ist.

3. Chirurgisches Instrument nach Anspruch 1, wobei die Datenkarte (410) an dem Batteriepaket (446) befestigt ist.

4. Chirurgisches Instrument nach Anspruch 1, wobei die Datenkarte (410) dafür konfiguriert ist, in einem Kartenleser (412), gesondert von dem Instrument, gelesen zu werden.

5. Chirurgisches Instrument nach Anspruch 4, wobei der Kartenleser (412) über eine drahtlose Verbindung in Kommunikation mit einem Rechner (414) steht.

6. Chirurgisches Instrument nach Anspruch 4, wobei der Kartenleser (412) dafür konfiguriert ist, ein drahtloses Signal, das Daten einschließt, an einen Rechner (414) zu übermitteln, wenn die Datenkarte (410) in dem Kartenleser aufgenommen wird.

7. Chirurgisches Instrument nach Anspruch 6, wobei die Daten eine Anzahl von Minuten umfassen, die das Instrument während eines Verfahrens verwendet wurde, wobei es ferner ein Elektronikmodul (413) in Kommunikation mit der Datenkarte (410) umfasst, wobei die Daten an einen Dritten übermittelbar sind, der mit dem Elektronikmodul verknüpft ist, um für die Verwendung des Elektronikmoduls während einer Operation abzurechnen.

8. Chirurgisches Instrument nach Anspruch 6, wobei die Daten eine Anforderung, eine Art von Operation zu erwerben, umfassen, wobei die Art von Operation eine zugehörige Anzahl von Minuten und einen Satz von Instrumentenparametern einschließt, und wobei die Datenkarte (410) dafür konfiguriert ist, Informationen bezüglich einer erworbenen Art von Operation über eine Erwerbstransaktion zu empfangen, so dass das Instrument für die zugehörige Anzahl von Minuten während der erworbenen Art von Operation verwendbar ist, wenn die Datenkarte aus dem Kartenleser (412) entfernt und in der Öffnung des Instruments aufgenommen wird.

9. Chirurgisches Instrument nach Anspruch 6, wobei die Daten eine Anforderung, eine Anzahl von Minuten zu erwerben, umfassen, und die Datenkarte (410) dafür konfiguriert ist, eine erworbene Anzahl von Minuten über eine Erwerbstransaktion auf der Grundlage der angeforderten Anzahl von Minuten zu empfangen, so dass das Instrument für die erworbene Anzahl von Minuten während einer Operation verwendbar ist, wenn die Datenkarte aus dem Kartenleser (412) entfernt und in der Öffnung (442) des Instruments aufgenommen wird.

10. Chirurgisches Instrument nach Anspruch 9, wobei der Rechner (414) dafür konfiguriert ist, die Daten über ein Netz zu einer äußeren Quelle zu senden, um die Erwerbstransaktion durchzuführen.

11. Chirurgisches Instrument nach Anspruch 1, wobei der Endeffektor (140) dafür konfiguriert ist, ein drahtloses Signal, das Daten einschließt, an wenigstens eines von einem Rechner (414) oder der Datenkarte (410) zu übermitteln.

12. Chirurgisches Instrument nach Anspruch 1, wobei die Datenkarte (410) dafür konfiguriert ist, ein drahtloses Signal, das Daten einschließt, an einen Rechner (414) zu übermitteln, wenn die Datenkarte in der Öffnung (442) des Instruments aufgenommen wird.

## Revendications

1. Instrument chirurgical comprenant :
(a) un ensemble bloc-batterie comportant une carte de données (410) et un bloc-batterie (446) ; ;
(b) un corps comprenant un boîtier (130) définissant une ouverture (442), dans lequel l'ouverture est configurée pour recevoir l'ensemble bloc-batterie ;
(c) un ensemble de transmission s'étendant de façon distale depuis le corps ; et
(d) un organe effecteur terminal (140) au niveau d'une extrémité distale de l'ensemble de transmission, dans lequel l'organe effecteur terminal est opérationnel pour distribuer de l'énergie de l'ensemble de transmission à un site chirurgical ;
dans lequel la carte de données est opérationnelle pour stocker des informations concernant un paramètre de fonctionnement associé à l'organe effecteur terminal ; et
dans lequel le boîtier comporte une portion inférieure et une pièce terminale, dans lequel la pièce terminale est fixée de façon articulée à la portion inférieure et est positionnable entre une position ouverte et une position fermée, et dans lequel l'ouverture est disposée au niveau d'une extrémité basse de la portion inférieure de sorte que l'ensemble bloc-batterie soit reçu de façon amovible dans l'ouverture lorsque la pièce terminale est dans la position ouverte et que l'ensemble bloc-batterie soit contenu dans l'ouverture lorsque la pièce terminale est dans la position fermée.

2. Instrument chirurgical selon la revendication 1, comprenant en outre un module électronique (413), dans lequel l'ouverture (442) est configurée pour recevoir le module électronique, dans lequel le bloc-batterie (446) est insérable dans le module électronique.

3. Instrument chirurgical selon la revendication 1, dans lequel la carte de données (410) est fixée au bloc-batterie (446).

4. Instrument chirurgical selon la revendication 1, dans lequel la carte de données (410) est configurée pour être lue dans un lecteur de cartes (412) séparé de l'instrument.

5. Instrument chirurgical selon la revendication 4, dans lequel le lecteur de cartes (412) est en communication avec un ordinateur (414) via une connexion sans fil.

6. Instrument chirurgical selon la revendication 4, dans lequel le lecteur de cartes (412) est configuré pour transmettre un signal sans fil comportant des données à un ordinateur (414) lorsque la carte de données (410) est reçue dans le lecteur de cartes.

7. Instrument chirurgical selon la revendication 6, dans lequel les données comprennent un nombre de minutes pendant lesquelles l'instrument a été utilisé durant une procédure, comprenant en outre un module électronique (413) en communication avec la carte de données (410), dans lequel les données peuvent être transmises à un tiers associé au module électronique pour facturer l'utilisation du module électronique durant une procédure.

8. Instrument chirurgical selon la revendication 6, dans lequel les données comprennent une demande d'achat d'un type de procédure, dans lequel le type de procédure comporte un nombre de minutes associé et un jeu de paramètres d'instrument, et dans lequel la carte de données (410) est configurée pour recevoir des informations concernant un type de procédure acheté via une transaction d'achat, de sorte que l'instrument soit utilisable pendant le nombre de minutes associé durant le type de procédure acheté lorsque la carte de données est retirée du lecteur de cartes (412) et reçue dans l'ouverture de l'instrument.

9. Instrument chirurgical selon la revendication 6, dans lequel les données comprennent une demande d'achat d'un nombre de minutes, et la carte de données (410) est configurée pour recevoir un nombre de minutes acheté via une transaction d'achat d'après le nombre de minutes demandé, de sorte que l'instrument soit utilisable pendant le nombre de minutes acheté durant une procédure lorsque la carte de données est retirée du lecteur de cartes (412) et reçue dans l'ouverture (442) de l'instrument.

10. Instrument chirurgical selon la revendication 9, dans lequel l'ordinateur (414) est configuré pour envoyer les données à une source extérieure via un réseau pour mener la transaction d'achat.

11. Instrument chirurgical selon la revendication 1, dans lequel l'organe effecteur terminal (140) est configuré pour transmettre un signal sans fil comportant des données à au moins l'un d'un ordinateur (414) ou de la carte de données (410).

12. Instrument chirurgical selon la revendication 1, dans lequel la carte de données (410) est configurée pour transmettre un signal sans fil comportant des données à un ordinateur (414) lorsque la carte de données est reçue dans l'ouverture (442) de l'instrument.
